# EUROPEAN PATENT SPECIFICATION

(11) **EP 0 899 326 B1**
(45) Date of publication and mention of the grant of the patent: **17.09.2003**
(21) Application number: 98116182.1
(22) Date of filing: 27.08.1998
(51) Int. Cl.: C12N 1/00

(54) **Inactivated micro-organisms containing digestive enzymes, process for their preparation, and their use in the food sector**
Verdauungsenzymen enthaltenden Inaktivierten Mikroorganismen, Verfahren zu deren Herstellung, und ihre Verwendung im Lebensmittelbereich
Micro-organismes inactivés contenant des enzymes digestifs, procédé pour leur préparation, et leur utilisation dans le domaine alimentaire

(30) Priority: 29.08.1997 IT MI971987
(43) Date of publication of application: 03.03.1999
(73) Proprietor: DOX-AL ITALIA S.p.A., I-20050 Correzzana Milano (IT)
(72) Inventor: Volpato, Ivo, 06070 S. Mariano (IT); Bizzini, Bernard, 46100 Figeac (FR); Veneroni, Flavio, 20050 Correzzana (IT)
(74) Representative: Gervasi, Gemma, Dr.

(56) References cited:
- EP-A- 0 085 805
- EP-A- 0 111 202
- EP-A- 0 242 135
- EP-A- 0 453 316
- FR-A- 2 179 528

## Description

### SCOPE OF INVENTION

The present invention relates to a new process for obtaining inactivated micro-organisms which may be advantageously used as vehicles of digestive enzymes and as probiotic agents, both for human consumption and for veterinary purposes, and in particular as components of premix and animal feed for rearing livestock.

### STATE OF THE ART

The digestive process has the function of transforming food products (proteins, carbohydrates and lipids) into single-structured substances that can be absorbed through the intestinal wall. This process essentially involves enzymes that can be produced by internal organs, such as salivary and pancreatic amylases, pepsin, intestinal proteases, saccharidases and lipases. Enzymes of the intestinal flora, such as cellulases, may moreover be involved in the digestive process. In fact, only herbivorous animal species are able to metabolize cellulose, and this is made possible precisely because of the particular type of the intestinal and ruminal flora.

Since absorption takes place only after the complete transformation of the food substances into their homostructural components, except in the case of lipids, the usefulness of a supply of digestive enzymes such as to guarantee the best conditions of catabolism of nutrient substances is evident. This need has to be considered borne in mind both in the case of dysfunction of secretory organs and in the case of reduced viability of the intestinal microflora, which are responsible for enzymatic biosynthesis. This latter case may frequently be found in the sector of zootechnics, often relying on antibiotic therapies for preventive purposes. This treatment is the cause of alterations, which may be even considerable, of the intestinal microbial ecosystem, with consequent reduction in endogenous enzymatic biosynthesis.

In the rearing of livestock, the optimization of the use of food by the animals themselves plays a fundamental role in the achievement of the maximum degree of competitiveness, having a considerable effect on the quality of the final produce (for example, meat and milk). In particular, in animal species that are bred, in view of the prevalently vegetal origin of the food base, the enzymes produced by the intestinal flora assume a fundamental importance.

In addition, in zootechnics, the optimization of the digestive process results in an improvement of the general state of health of the animal, in a significant reduction in costs, as well as in a reduction of the environmental impact deriving from organic waste. In fact, in pig-rearing, for example, on account of the lack of phytases, diets particularly rich in phosphates are used, these substances being one of the major sources of environmental pollution deriving from such breeding activities.

Given the importance of a proper intestinal enzymatic balance and the influence that this may have on animal breeding, the need for an exogenous enzymatic supply is warranted, in particular in the animal species that are subject to hyperfeeding and intensive pharmacological treatment for preventive purposes, as referred to above.

This result may be obtained by associating appropriate enzymes, such as cellulases, phytases, proteases, lipases, etc., to the normal livestock animal feed. The presence of enzymes in optimal quantities induces, in fact, an increase in the digestive process, facilitating the assimilation of the essential macro- and micro-molecules.

However, the methods for administering enzymes known today in the state of the art are limited by the problems linked to the easy biological inactivation of such structures of a protein nature, which are characterized by high structural instability, being particularly sensitive to chemical and physical-chemical environmental variations, such as pH, temperature, light, mixing with other components, presence of reaction substrates, presence of proteolytic enzymes, etc. For this reason, their use calls for particular methodologies and precautions with the aim of preserving their original properties unaltered.

In the state of the art, the administration of enzymes in a protected form is known, for example, in the form of gastro-protected granules or in the form of extract, in so far as the simultaneous presence of specific inhibitors is able to reduce advantageously the kinetics of enzymatic degradation. The use of enzymes that have undergone inert-phase immobilization is moreover known.

However, the above-mentioned methodologies reveal various disadvantages, such as:
- the treatments required may induce inactivation of the enzyme;
- high costs, which are far from compatible with extensive applications, for example in the sector of zootechnics;
- difficulty of use in the animal feed industry on account of the conditions of physical-chemical stress usually present in the processes of preparation and mixing of the feeds.

There is therefore felt the need for a new method of enzymatic administration which is convenient and economic, both in the human field and for veterinary purposes, such as may be advantageously proposed on a large scale at industrially accessible costs.

### SUMMARY

Object of the present invention is a process for producing inactivated micro-organisms containing digestive enzymes, comprising the following steps:
1) extraction of the endocellular mass from the cells of an appropriate micro-organism by means of hyperosmotic treatment, and separation of the extracted endocellular mass;
2) possible inactivation of the micro-organism obtained in step (1) by chemical or physical means, leaving the external membrane of the micro-organism unaltered;
3) intracellular insertion of one or more digestive enzymes in the inactivated micro-organism obtained in step (1) or (2) by means of hypo-osmotic treatment, by treating the said micro-organism in a hypotonic suspension containing the endogenous material that has been extracted in step (1), to which are possibly added digestive enzymes of another nature and other essential nutrients, or in a suitable hypotonic aqueous suspension containing said digestive enzymes and other essential nutrients.

The present invention moreover regards the inactivated micro-organisms containing digestive enzymes, obtained with the process described above, the compositions containing them, possibly in association with appropriate food, excipients and/or diluents, as well as their use in food, both in the human field and for veterinary purposes, and in particular in zootechnics as components of animal feed and premix for livestock.

### DETAILED DESCRIPTION OF THE INVENTION

The characteristics and advantages of the process for the production of inactivated micro-organisms enriched with digestive enzymes, of the inactivated micro-organisms thus obtained, of the compositions containing them, and their use in the human or veterinary food sector, according to the present invention, will be more fully illustrated in the course of the detailed description that follows.

The present invention enables the administration of digestive enzymes incorporated inside the cellular walls of micro-organisms that have been inactivated, i.e., "killed", which are able to protect the structural characteristics and activity thereof.

The micro-organisms used in the process according to the present invention must possess good characteristics for serving as vehicles, i.e., good resistance to chemical and physical-chemical stress. The said micro-organisms may be selected also on the basis of the characteristics of affinity and tolerability in regard to the host cells.

These micro-organisms may be:
(i) micro-organisms not producers of specific digestive enzymes, used after inactivation as vehicles of one or more digestive enzymes;
(ii) micro-organisms that are producers of one or more specific digestive enzymes;
(iii) micro-organisms that are not producers of specific digestive enzymes, but that may be conditioned to produce such enzymes.

In all three of the above cases, the micro-organisms may be used for transporting enzymes whether produced or not produced by the micro-organisms themselves.

In case (i), the strains of micro-organisms, used as transporters of enzymes of animal or vegetal origin, must have appropriate characteristics of resistance, in that they must be able to reach the intestine structurally intact.

Amongst the known strains available on the market, the *Saccharomyces cerevisiae* is preferred, which has characteristics of structural resistance to the attack of chemical and physical-chemical agents, and is able to reach intact the site in which the intestinal digestive process takes place. The said micro-organism moreover performs an advantageous probiotic activity, as described in the European patent No. EP 0 111 202.

Before the emptying-out phase, the micro-organisms may be made to grow in appropriate fermenters, according to the methods and conditions known in the state of the art, the micro-organic mass being separated from the culture medium, at the end of the growth phase, by means of filtration or centrifugation.

In **case (ii)**, it is possible to use micro-organisms that are known for the production of specific digestive enzymes, which normally belong to the intestinal microflora (e.g., *Bacillus subtilis* and *Lactobacillus sp.)* and are readily available on the market. The capacity of the said micro-organisms to produce enzymes may be previously verified by means of techniques known in the state of the art.

Furthermore, the said micro-organisms may be conditioned, according to methods known in the state of the art for the purpose of optimizing the industrial yields of the enzyme produced.

Before the emptying-out step (1) of the process of the invention, the fermentative growth of the micro-organism is obtained, with the production of digestive enzymes, according to the batch or continuous techniques commonly used in the state of the art, for example in the operating conditions referred to in *Biochemical Engineering and Biotechnology Handbook,* B. Atkinson and F. Mavituna Eds., 1991, MacMillan Publ., Chapter 6.7.

The micro-organisms may be separated from the growth medium by filtration or centrifugation, recovering the supernatant in the case where the enzymes of interest are exo-enzymes (i.e., released in the growth medium). The supernatant is then concentrated, and the enzymes of interest may be recovered by saline precipitation with ammonium sulphate, as described by Robert K. Scopes in *Protein Purification, Principles and Practice,* Chap. 3, Pages 39-52, Springer-Verlag Inc., New York, 1982. The protein precipitate may then be separated by filtration, re-suspended in water and subjected to dialysis with molecular cut-off at 2,000/5,000 daltons against water for 48 hours, said operations being carried out under cooling. This precipitate, the specific enzymatic activity of which may be verified using standard methods (see, for example, A.J. Pesce and L.A. Kaplan, *Methods in Clinical Chemistry,* The C.V. Mosby Company, 1987, pages 817-821 for amylases; pages 848-853 for lipases; pages 857-861 for trypsin), may be subsequently used in the re-incorporation step (3).

The degree of purity of the proteins thus obtained may be evaluated by means of control of electrophoretic mobility, as described by Robert K. Scopes in *Protein Purification, Principles and Practice,* Chap. 9, Pages 245-254, Springer-Verlag Inc., New York, 1982.

The micro-organisms used in **case (iii)** are preferably "vulgar" strains, isolated from human faecal material and belonging to the so-called "good" intestinal flora, or by ruminal, intestinal or faecal material of the "good" intestinal flora and of other sources of the different species of livestock. Isolation is carried out according to methods known in the state of the art for the development of microflora in nutrient culture and subsequent selection of the colonies on the basis of their morphological and taxonomic characteristics. The characterization of the isolated strains is according to methodologies commonly used, for example by analysis of Gram's stain, metabolic behaviour, production of characteristic chemical products, and nutritive characteristics.

The aforementioned strains, which initially are not able to produce the desired enzyme, are subjected to a process of conditioning for the production of specific enzymes, using methods commonly employed in the state of the art. The conditioning is carried out by gradual enrichment of the growth medium of the micro-organism with the substrate metabolized by the enzyme to be produced, in concentrations that increase in a scalar fashion, and by reducing at the same time the other sources of elements present in the nutrient culture, until said material represents the essential source of energy for the micro-organism. In this way, the micro-organisms are induced to produce the enzymes involved in the metabolization of this material, which constitutes the prevalent source of nutrition for their growth and development.

With the increase in the culture medium of the material to be metabolized, there is noted a reduction in the number of micro-organisms that are able to reproduce in such conditions, until the selection of the conditioned strains is obtained that are suitable for the production of specific enzymatic systems, which may be characterized and possibly isolated on the basis of their taxonomic properties. In general, at least 36-72 growth cycles are required to obtained the above-mentioned result.

For example, in the case of micro-organisms that produce cellulases, these are conditioned to the use of substrates, such as cellulose and phytates as prevalent source of essential food nutrients (C, N, and O).

The control of the production of the desired enzymes is made by means of an analysis of the protein content, for instance according to the biuret method (SIGMA kit, Code 690-A, 1992 catalogue), and of the specific enzymatic activity of the growth medium, according to methods known in the state of the art, in the case where the enzymes produced are exo-enzymes. In the case, instead, of endo-enzymes, the aforesaid determinations of activity must be preceded by an analysis of the endocellular content of an aliquot of the micro-organisms by means of "squeezing" in a hypertonic medium.

After the conditioning step, also these micro-organisms are subjected to a process of fermentation, as described above, to produce the desired enzymes, before proceeding to the emptying-out step (1).

The digestive enzymes vehicled using the process of the invention may be endo-enzymes, which remain inside the micro-organism, or exo-enzymes, which are released into the growth medium. These enzymes are preferably selected from the group consisting of amylases, saccharidases, lipases, proteases, pectinases, cellulases, and peptidases (carboxypeptidases, aminopeptidases, dipeptidases, and tripeptidases).

For the production of amylases and saccharidases, the strains that produce these belong preferably to the species *Acinetobacter*, *Bacillus, Aspergillus,* and *Endomyces,* and in particular are chosen from the group consisting of *Bacillus subtilis*, *Bacillus* cereus, *Bacillus* licheniformis, *Bacillus amyloliquefaciens, Bacillus* polymyxa, *Aspergillus niger, Aspergillus oryzae,* and *Micrococcus halobius.*

For the production of lipases, micro-organisms of the species *Lactobacillus, Micrococcus* and *Aspergillus* are preferably used, and in particular they are chosen from the group consisting of *Aspergillus niger, Aspergillus oryzae* and *Lactobacillus acidofilus.*

For the production of proteases, which may be differentiated according to the amino-acid bond site of interaction, micro-organisms of the species *Pseudomonas, Streptomyces* and *Bacillus* are preferably used, and in particular they are chosen from the group consisting of *Bacillus* subtilis, *Bacillus licheniformis, Bacillus thermoproteolyticus, Aspergillus niger, Aspergillus oryzae, Aspergillus melleus* and *Escherichia coli.*

For the production of pectinases, micro-organisms of the species *Acrocylindrum, Aspergillus* and *Penicillium* are preferably used, and in particular they are chosen from the group consisting of *Aspergillus niger, Aspergillus alliaceus, Aspergillus flavus, Aspergillus oryzae, Fusarium oxysporum, Clostridium multifermentans, Bacillus subtilis* and *Pseudomonas fluorescens.*

Further micro-organisms that may be advantageously used in the process of the invention, as well as some of their sources, are reported in *Biochemical Engineering and Biotechnology Handbook,* B. Atkinson and F. Mavituna Eds., 1991, MacMillan Publ., Chapters 6.1, 6.7 and 9.5.

Furthermore, micro-organisms that produce cellulases may be advantageously used, among which endoglucanases, cellobiohydrolases and betaglucosidases.

These micro-organisms may be bacteria of the species *Ruminococcus, Cellulomonas* and *Pseudomonas,* and in particular *Pseudomonas fluorescens;* fungi of the species *Penicillium, Aspergillus* and *Trichoderma*, and in particular *Penicillium* funiculosum, *Aspergillus niger, Trichoderma reesei, Trichoderma viride* and *Trichoderma koniugii;* actinomycetes of the species *Streptomyces* and *Thermoactinomyces.*

In the emptying-out **step (1)**, the endocellular mass is caused to come out of the micro-organic cellular walls by means of the hyperosmotic treatment of "squeezing" of the micro-organisms obtained as described above. In the case where the micro-organisms are previously subjected to fermentation with the purpose of producing the desired digestive enzymes, the latter are taken off at the maximum peak of the cellular growth curve, before start of the phase of stasis.

Step (1) is carried out by suspension of the micro-organic mass in a hypertonic aqueous solution containing:
- NaCI in concentrations of over 0.2-0.3 M;
- sodium citrate in concentrations of over 0.05 M;
- an appropriate antibacterial agent, preferably thimerosal (in particular Merthiolate® , 1995 Sigma catalogue), in suitable concentrations.

Preferably the aforesaid hypertonic solution contains NaCl 1 M, sodium citrate 0.1 M, and thimerosal 0.1 mg/l.

The suspension thus obtained is then kept under agitation at a temperature ranging between 2 and 8°C, preferably 4°C, for a period of between 2 hours and 4 days, preferably 72 hours, obtaining the squeezing of the micro-organisms as the endocellular content is extruded into the hypertonic medium. The endocellular mass which is caused to come out of the cells may comprise also the digestive enzymes produced. In fact, in this way it is possible to safeguard the chemical structure of the enzymes, which would be irremediably damaged in the possible subsequent step of inactivation of the micro-organism (step 2).

The micro-organisms thus emptied out and reduced to their cellular walls alone are smaller than normal, and the membrane pores are enlarged. The micro-organisms may be separated from the endocellular mass that has come out in the suspension medium using techniques known in the state of the art, and preferably by means of filtration or centrifugation.

The control of the emptying-out of the micro-organic walls may be conveniently carried out by microscopic evaluation of the morphology of the walls themselves, which must appear smaller and wrinkled.

Once the emptying-out step is terminated, the suspension medium (i.e., the hypertonic buffer and the endocellular content that has been extacted) may be separated from the emptied micro-organisms by means of centrifugation at 8,000-15,000 r.p.m., preferably 10,000 r.p.m. In the case of the micro-organisms (ii) and (iii), the suspension medium is appropriately treated for the recovery of the enzymes contained therein and for the elimination of the saline hyperconcentration. In particular, this medium may be dialysed with molecular cut-off at 2,000 daltons, and the enzymatic content may be isolated by saline precipitation with ammonium sulphate, as described above. The precipitate thus obtained may then be re-suspended in water, dialysed with the purpose of eliminating the ammonium sulphate, and finally used for the preparation of the hypotonic medium used in step (3).

In **step (2)**, the micro-organisms which have been emptied of their endocellular mass may be inactivated, i.e., killed, by means of suitable chemical or physical treatment. Such treatment may not be necessary for certain micro-organisms, in so far as the hypertonic treatment of step (1) in many cases already proves sufficient to inactivate the cells. Hence, this step (2) may be carried out after the state of inactivation of the micro-organisms obtained from step (1) has been verified.

Among the chemical methods preferably used for micro-organisms having cellular walls that are easily degradable to heat, treatments using disinfectant substances (with concentrations of 0.05-0.2 mg/l) may be employed for a period of 2-12 hours, and preferably with thimerosal, at a concentration of 0.1 mg/l, for a period of 6-8 hours.

This chemical inactivation may be performed also during the previous emptying-out step, by adding the aforementioned disinfectant substances to the hypertonic solution.

Physical treatments preferably comprise exposure to UV rays and thermal inactivation by heating of the suspension obtained from step (1) at a temperature ranging between 55 and 65°C, preferably 60°C, and finally isolating the inactivated micro-organisms by concentration and filtration.

This treatment may be carried out also at the start of step (3) of intracellular re-integration in hypotonic medium. In this case, the emptied micro-organisms obtained from step (1) are suspended in a part of the hypotonic medium containing the enzymes to be re-incorporated, and the mixture thus obtained is then heated up in the conditions referred to above. After cooling to 2-8°C, preferably 4°C, the remaining part of the hypotonic medium is added, and the next step of intracellular re-integration proceeds as described in step (3).

The conditions of inactivation must be such as not to cause alterations in the parietal structure of the micro-organism, and hence must be chosen according to the characteristics of resistance of the micro-organism itself. The control of the degree of inactivation of the micro-organic cells at the end of step (2) may be carried out using culture tests, as follows: a small aliquot of treated micro-organic cells are re-suspended in part of the previously extracted endocellular mass, after dialysis of the same for elimination of the salts and after having re-established isotonicity. When the cells have re-acquired their original shape, approximately 1 g or 1 ml of their suspension is inoculated in a selective growth medium (approx. 15-25 ml of nutrient broth), and the cells are left in incubation (at approx. 37°C for bacteria; at approx. 25°C in the case of Mycetes) for a period of 36-48 hours.

Since this first stage may not be sufficient to restore totally viability to the micro-organism, the sowing may be repeated for further subsequent transplants (preferably 3), using the broth obtained from the first step. Possible processes of growth are controlled by analysing the concentration of the colonies present by means of turbidimetric analysis, or under the microscope (on slides after staining), or else by using colony counters.

In **step (3)**, there is the intracellular integration, in the inactivated micro-organic cells, of one or more digestive enzymes to be vehicled, by means of treatment of said inactivated cells in a hypotonic aqueous solution containing said enzymes, under gentle stirring, for a period sufficiently long to enable re-incorporation of the material in the cells, i.e., preferably between 4 hours and 4 days, even more preferably 72 hours, at a temperature ranging between 2 and 8°C, preferably of 4°C.

The aforesaid hypertonic solution preferably contains:
- NaCI in concentrations of less than 0.12 M;
- sodium citrate in concentrations of less than 0.025 M;
- an appropriate antibacterial agent, preferably thimerosal (in particular Merthiolate®, 1995 Sigma catalogue) in appropriate concentrations.

Preferably the aforesaid hypertonic solution contains NaCI 0.05 M, sodium citrate 0.005 M, and thimerosal 0.1 mg/l.

During the operation of intracellular re-integration, the biomass and the enzymatic material contained in the hypotonic suspension are absorbed within the inactivated micro-organisms, which re-acquire their original shape. The control of the re-absorption may be conducted evaluating the quantity of enzymes still present in the hypotonic aqueous suspension, or by means of lysis of certain of the micro-organic cells and through determination of the enzymatic activity of the lysate.

In the case of cells used only as vehicling agents (case (i)), the inactivated micro-organisms may be immersed in a hypotonic suspension medium containing one or more digestive enzymes, of animal or vegetal nature, not produced by the micro-organisms used (hence not contained in the endocellular mass extracted), with the possible addition of other essential nutrients, such as vitamins and glucides.

In the case, instead, where the micro-organisms (ii) and (iii) are used, said aqueous suspension may consist of the endocellular mass extracted in step (1) containing the digestive enzymes previously produced by the micro-organism, with the possible addition of other digestive enzymes of interest and essential nutrients, such as vitamins and glucides. The suspension medium containing the endocellular mass obtained in step (1) is previously subjected to dialysis for the purpose of eliminating the saline hyperconcentration due to the previous hyperosmotic treatment and to restore the suspension medium to conditions of isotonicity

In the case where the enzymes produced by the micro-organism are exo-enzymes, the suspension medium may consist either of enzymes recovered from the fermentation broths of the previous fermentation phase, as described above, or of the endocellular mass squeezed out in step (1). Differently, in the case of endo-enzymes, only the aforesaid endocellular mass is used.

The micro-organisms obtained from stage (3) may finally be concentrated, preferably by means of filtration, until small volumes of hypotonic medium are obtained, or they may be used directly in the preparation of food compositions. Such micro-organisms serve as excellent vehicles, at an intracellular level, of the digestive enzymes themselves and enable their administration in food both for humans and for veterinary purposes. These micro-organisms are particularly useful in zootechnics in that they work synergistically with the endogenous enzymes on the process of digestion of the various nutrients.

After intake of the micro-organisms of the invention by humans or animals, through the food or feed, in the phase of metabolization at the release level the fragmentation of the cell walls of the micro-organisms leads to the release of the digestive enzymes incorporated in the enriched micro-organisms. Since the enzymes thus protected do not come into contact with external inactivating factors, they maintain their original properties unaltered.

These micro-organisms not only perform a role of vehicles at an intestinal level, but also possess a probiotic function in so far as they lead to an improvement in the general state of health of the animal, and consequently in the quality of the produce to be consumed (milk, eggs, meat, etc.). In particular the micro-organisms of the invention show the following pharmacological effects:
- probiotic function: the non-polymeric fragments of the micro-organism, obtained from the fragmentation which occurs in the animal at the intestinal level, have a nutritive value;
- improvement in the conditions of growth of the so-called "good" intestinal microflora, such as bifidobacteria and lactobacillus;
- immunostimulant effects: in fact, certain components of the micro-organic membrane, such as glycoproteins and peptidoglycans, are able to stimulate the immunoprotective reactions of the animal itself;

The above-mentioned effects enable surprising advantages to be achieved, which include:
- improvement of the digestive process, with greater absorption of nutrients;
- improvement of the immune response of the animals to infectious illnesses, with a reduction in the quantities of antibiotics that need to be used to prevent illness;
- improvement of the growth curves of the animals and consequent reduction in breeding costs.

Finally, the inactivated micro-organisms according to the present invention offer the advantage of enabling homogeneous dispersion of the digestive enzymes in the feed, thus avoiding undesired accumulation of these substances and enabling administration of optimal, controllable and reproducible quantities of digestive enzymes in livestock animals.

Thus a further object of the present invention is the use of the aforesaid inactivated micro-organisms containing digestive enzymes in the food sector, both for human and for veterinary purposes, and preferably in zootechnics, in the rearing of livestock.

Finally, a further object of the present invention is represented by food compositions, usable in food for both human and veterinary purposes, comprising one or more inactivated micro-organisms enriched with digestive enzymes, as described above, in association with conventional foods, and possibly in the presence of appropriate excipients and/or diluents. The quantity of micro-organisms to be administered varies according to the animal species treated, the diet followed, the general state of health of the animals, and the conditions of rearing, and is preferably between 0.1 and 20 g per kg of feed for livestock, and from 0.1 to 5 g/day in food for human use. In the case of chickens, the micro-organisms of the invention are preferably administered in quantities of between 30 and 70 mg/day.

The above-mentioned compositions are preferably used in zootechnics in the form of premix or feed. In fact, the cellular walls of the micro-organism are able to protect the structural characteristics and activity of the enzymes contained therein both in the phase of preparation of the mixture and during its passage through the gastro-intestinal system.

To provide an illustration of the present invention, the following non-limitative example is given.

### EXAMPLE 1

### Preparation of inactivated Saccharomyces cerevisiae containing α-chymotrypsin, according to the present invention.

Cells of *Saccharomyces cerevisiae*, isolated from by-products of the processing of sugars using ordinary laboratory techniques, were made to grow by fermentation on cane sugar molasses in a 75-m³ fermenter, with aeration of 2 mM 02/l• min, under agitation of vvm, at a temperature of 30°C, with a pH of 4.5-5.5, and a culture density of 40-45 g/l. The cells were then separated by means of centrifugation at 10,000 r.p.m. for 15 minutes to obtain 0.5 g of dry product (corresponding to 15 •10⁹ cells) per 1 g of original substrate.

1kg of the cells of *Saccharomyces cerevisiae* thus obtained, was suspended in 25 I of a hypertonic aqueous solution containing NaCl 1 M, sodium citrate 1 M, and Merthiolate® 0.1 mg/l (1995 Sigma catalogue), and the suspension was kept under gentle stirring for 72 hours, at a temperature of 4°C. The suspension thus obtained was then centrifuged at 10,000 r.p.m. for 15 minutes, and the cells were recovered.

A quantity of 25 of a hypotonic aqueous solution containing NaCI 0.05 M, sodium citrate 0.005 M, and Merthiolate® 0.1 mg/l (1995 Sigma catalogue) was prepared.

This solution was cooled down to 4°C, and 25.6 g of the enzyme α-chymotrypsin, type I-S, obtained from bovine pancreas (40-60 units/mg; Code C 7762; 1992 Sigma catalogue) were added, and the mixture was kept under gentle agitation until complete solubilization.

In this hypotonic solution containing the enzyme to be introduced into the inactivated micro-organisms, were suspended the cells of *Saccharomyces cerevisiae* obtained as described above; the mixture was maintained under gentle stirring for a period of 72 hours, at a temperature of 4°C. Absorption of the enzyme was verified by analysing the protein content of the hypotonic medium, applying Lowry's method (Sigma kit Code P5656, 1992) and by analysis of the endocellular content of a small aliquot of treated micro-organic cells, determining the specific enzymatic activity of the lysate after the breaking down of said cells.

At the end of the intracellular introduction of the enzyme, the suspension was centrifuged at 10,000 r.p.m. for 15 minutes. The micro-organic cells were then collected and washed three times with a sterile hypotonic solution (volume ratio 1:10), leaving the suspension under gentle stirring for each washing, for 1-2 hours, and subsequently centrifuging in the conditions indicated above. The cells were finally collected in a small volume of sterile hypotonic solution (approximately 4-5l).

### BIOLOGICAL ACTIVITY

### (i) Analysis of growth curves, food consumption and digestive process in animals treated with the inactivated micro-organisms containing digestive enzymes according to the present invention.

Two groups of 30 rabbits in the growth phase, and two groups of 30 rats in the growth phase, belonging to both sexes, underwent treatment with food enriched with inactivated cells of *Saccharomyces cerevisiae* containing α-chymotrypsin, prepared according to Example 1, for a period of 45 days for the rats and 90 days for the rabbits, according to the following scheme:
Group 1:15 males and 15 females, with free access to food consisting of ordinary feed;
Group 2: 15 males and 15 females, with free access to food consisting of the feed referred to above, enriched with the micro-organisms according to Example 1, in the quantity of 5 g per kg of feed.

The general state of the animals under examination was controlled every day, and every 7th day the body weight trend and food consumption was evaluated.

Table 1 gives the percentage differences, as compared to the controls, for the two animal species tested at the end of the treatment period.

**Table 1**

| **Animal** | **% variation with respect to the controls** | |
|---|---|---|
| | **Body weight** | **Food consumption** |
| Male rat | +24.5 | -7.3 |
| Female rat | +17.7 | -5.7 |
| Male rabbit | +21.9 | -6.5 |
| Female rabbit | +18.5 | -6.0 |

The results given above show a significant increase in body weight in the animals treated with the inactivated micro-organisms containing digestive enzymes, according to the present invention, with respect to the control animals, in the presence of a simultaneous reduction in food consumption. These data indicate a greater utilization of the food and an improved bio-avialability of the homo = structural nutrients.

At the end of the treatment described above, the animals were sacrificed for the anatomical and pathological control of the state of the gastro-intestinal mucous membrane, and no significant alteration was found.

With the purpose of verifying also the level of absorption of the food consumed, the faeces of the above groups of animals were collected in the 24 hours before the end of treatment, with the aim of examining the modalities of elimination of one of the essential nutrients (i.e., proteins), taken as a reference parameter for an evaluation of the differences between the treated animals and the controls.

For each animal, 10 g of faecal material were suspended in 50 ml of PBS buffer at pH 7.0. The mixture was finely homogenized and kept under agitation for approximately 60 minutes, which was sufficient for the release of the protein, peptide and amino acid material into the aqueous medium.

The mixture was then centrifuged at 10,000 r.p.m. for 10 minutes; the supernatant was recovered and concentrated to a volume of 10 ml on a rotary vacuum evaporator at a temperature of 37-45°C.

On a first aliquot of 1 ml of concentrate, the presence of amine groups was evaluated as an index of the total content of protein nitrogen.

Further aliquots of 1 ml of concentrate were, instead, subjected to chromatographic analysis as an index of evaluation of the amount of digestive protein catabolism, as will be described later.

The determination of the amine groups was made according to the following method: to 1 ml of concentrate were added 0.5 ml of a solution of sodium tetraborate 0.1 M in NaOH 0.1 N. After addition of 20 µl of a solution of tetrahydrobenzene sulphonic acid 1.1 M, the mixture was stirred thoroughly and left to react for 5 minutes. At the end of this period, the reaction was blocked by adding 2 ml of a solution of NaH₂PO₄ 0.1 M containing sodium sulphite 1.5 mM.

The stain was detected by UV analysis at 420 nm by comparison with white.

Table 2 gives the percentage difference of the quantity of amines detected in the faeces of the animals treated with the inactivated micro-organisms enriched with digestive enzymes according to the invention, as compared to the control animals.

**Table 2**

| **Animal** | **Amine % variation with respect to the controls** |
|---|---|
| Male rat | -12.4 |
| Female rat | -10.6 |
| Male rabbit | -13.5 |
| Female rabbit | -9.7 |

The data tabulated above show that the administration of the micro-organisms according to the present invention causes a reduction in amine groups in faeces.

In percentage terms, this reduction is higher than the reduction in the amount of food intake, thus witnessing to an improved absorption of the nitrogenated nutrients.

Furthermore, as mentioned previously, aliquots of 1 ml of the concentrate underwent chromatographic analysis with the aim of evaluating the distribution of the molecular weight (MW) of the protein material and hence of obtaining an indirect evaluation of its degree of degradation during the digestive process.

Aliquots of 1 ml of concentrated faecal extract of each male animal, prepared as described previously, underwent gel-filtration chromatography on Sephadex G15, G25 and G200, according to the operating instructions described in *Gel-filtration, Theory and* Practice (Pharmacia, pages 7-11, 1985-86) and in *Protein Purification,* Principles *and Practice* (Robert K. Scopes; Chap. 5, pages 151-163, Springer-Verlag Inc., New York, 1982). The individually obtained eluates were then collected and concentrated to a volume of 1 ml using a rotary vacuum evaporator at a temperature of 37-45°C. For each of the samples obtained, the peptide/protein content was determined using Lowry's method (Sigma kit P-5656, 1992), which is particularly useful also in detecting low-MW peptide fragments.

Table 3 shows the percentage variations of the quantities of peptides and proteins, in precise MW ranges, contained in the faeces of the animals examined.

**Table 3**

| **Animal** | **Type of Sephadex** | **MW range** | **% variation in peptide/protein content in faeces** |
|---|---|---|---|
| Male rat | G15 | 50-1,500 | +25.9 |
| Male rabbit | G15 | 50-1,500 | +23.7 |
| Male rat | G25 | 1,000-5,000 | +16.2 |
| Male rabbit | G25 | 1,000-5,000 | +18.7 |
| Male rat | G200 | 5,000-600,000 | -36.0 |
| Male rabbit | G200 | 5,000-600,000 | -35.4 |

The increase in the concentration of low-molecular weight fragments and the simultaneous reduction in high-molecular weight fragments demonstrate an increase of the enzymatic activity during the digestive process in the animals treated with the micro-organisms according to the present invention.

### (ii) Influence of the micro-organisms on experimental induction of infectious diseases

The following tests were conducted on 4 groups of 20 male Swiss mice, weighing 20±2 g, which were treated, in the 20 days preceding induction of the experimental infection, with ordinary food (controls) or with food enriched with the micro-organisms of the invention, and in particular fed with the same feed as the controls, enriched with 5 g of micro-organisms prepared as described in Example 1 per kg of feed.

The animals were then infected, via intranasal route, with *K. pneumoniae* (strain 52145 PITC) or with influenza virus (strain A/PR8/34) so as to cause a mild infection, according to the method described by M. Fattal-German and B. Bizzini (*Develop. Biol. Standard,* 77:115-120 and 137-142, 1992). Following on inoculation, the animals were again fed as described above, i.e., with ordinary feed or with feed enriched with the inactivated micro-organisms of the invention. The survival percentage after given time intervals was evaluated according to the following scheme:
Group 1 (controls): after 20 days of ordinary feeding, intranasal inoculation with *K.* *pneumoniae* and check on number of animals surviving after 7 days of infection;
Group 2 (controls): after 20 days of ordinary feeding, intranasal inoculation with influenza virus, strain A/PR8/34, and check on number of animals surviving after 18 days of infection;
Group 3: after 20 days of diet enriched with the micro-organisms of the invention, as described above, intranasal inoculation with *K. pneumoniae* and check on number of animals surviving after 7 days of infection;
Group 4: after 20 days of ordinary diet enriched with the micro-organisms of the invention, as described above, intranasal inoculation with influenza virus, strain A/PR8/34, and check on number of animals surviving after 18 days of infection.

Table 4 gives the percentage survival rates of the animals belonging to the groups referred to above, after the specified time intervals.

**Table 4**

| **Group** | **Diet** | **Type of infection** | **% of animals surviving** |
|---|---|---|---|
| 1 | normal | *K. pneumoniae* | 10 |
| 2 | normal | Influenza virus | 30 |
| 3 | enriched | *K. pneumoniae* | 25 |
| 4 | enriched | Influenza virus | 60 |

The results reported above witness to the fact that there is an increase in defences against infectious agents in the animals treated with the micro-organisms according to the present invention as compared to the controls. This increase may related to an improvement in the state of health of the animals, and, above all, to an increase in the immune defences in regard to attack by pathogenic agents.

### (iii) Evaluation of the stimulation of the activity of the reticulo-endothelial system by the micro-organisms of the invention, as an index of their immunostimulant activity

The following tests were carried out on 2 groups of 20 male Swiss mice, weighing 20±2 g each, treated, in the 20 days leading up to the test, either with normal food (Group 1, controls) or with food enriched with the micro-organisms of the invention, and in particular fed with the same feed as the controls, enriched at each intake of food with 5 g of the micro-organisms prepared according to Example 1 per kg of feed (Group 2, test group).

The animals were then treated, intravenously, with 24 mg/100 g of animal weight of carbon particles (Indian ink), according to the method described by B.N. Halpern *et al.* (*Annales de l'Institut Pasteur,* 80(6):582-604,1951). In this way, the rate of elimination of the injected carbon particles from the blood circulation was evaluated. The percentage reduction in the circulating particles was evaluated using turbidimetric techniques after 30 minutes from intravenous injection of the ink.

The animals of Group 2, i.e., the ones treated with the micro-organisms according to the present invention, showed a percentage increase in elimination of the particles of 32.5% with respect to the animals of Group 1, i.e., those receiving a normal diet. This provides an evident demonstration of the immunostimulant effect induced by the food regime enriched according to the present invention.

## Claims

1. Process for producing inactivated micro-organisms containing one or more digestive enzymes, which comprises the following steps:
1) extraction of the endocellular mass from the cells of an appropriate micro-organism by means of hyperosmotic treatment, and separation of the extracted endocellular mass;
2) possible inactivation of the micro-organism obtained in step (1) by chemical or physical means, leaving the external membrane of the micro-organism unaltered;
3) intracellular insertion of one or more digestive enzymes in the inactivated micro-organism obtained in step (1) or (2) by means of hypo-osmotic treatment, by treating said micro-organism in a hypotonic suspension containing the endogenous material that has been extracted in step (1), to which are possibly added digestive enzymes of other nature and other essential nutrients, or a suitable hypotonic aqueous suspension containing said digestive enzymes and other essential nutrients.

2. Process according to Claim 1, **characterized in that** said micro-organism is not a producer of digestive enzymes, but acts as vehicle of said enzymes.

3. Process according to claim 2, **characterized in that** said microorganisms is *Saccharomyces cerevisiae.*

4. Process according to Claim 1, **characterized in that** said micro-organism is a producer of one or more specific digestive enzymes chosen from the group consisting of amylases, saccharidases, lipases, proteases, pectinases, cellulases and peptidases.

5. Process according to Claim 4, **characterized in that**, when said digestive enzymes are amylases or saccharidases, the said micro-organism belongs to the species *Acinetobacter, Bacillus, Aspergillus, Micrococcus o Endomyces.*

6. Process according to Claim 4, **characterized in that**, when said digestive enzymes are lipases, the said micro-organism belongs to the species *Lactobacillus, Micrococcus,* Aspergillus, *Pseudomonas* o *Penicillium.*

7. Process according to Claim 4, **characterized in that**, when said digestive enzymes are proteases, the said micro-organism belongs to the species *Pseudomonas, Streptomyces, Bacillus* or *Aspergillus.*

8. Process according to Claim 4, **characterized in that**, when said digestive enzymes are pectinases, said micro-organism belongs to the species *Acrocylindrum, Aspergillus,* Penicillium, Fusarium, Clostridium, *Bacillus* or *Pseudomonas.*

9. Process according to Claim 4, **characterized in that**, when said digestive enzymes are cellulases, said micro-organism is a bacterium of the species *Ruminococcus, Cellulomonas* or *Pseudomonas,* or else a fungus of the species *Penicillium, Aspergillus* or *Trichoderma*, or else an actinomycete of the species *Streptomyces* or *Thermoactinomyces.*

10. Process according to Claim 1, **characterized in that** said micro-organism is not a producer of digestive enzymes, is isolated from human faecal material or ruminal, intestinal and/or faecal material of livestock animals, and is appropriately conditioned to the production of said enzymes.

11. Process according to Claim 1, **characterized in that**, before step (1), said micro-organisms are made to grow by fermentation, possibly recovering the digestive enzymes produced during said fermentation.

12. Process according to Claim 1, **characterized in that** said hyperosmotic treatment consists in suspending the said micro-organism in a hypertonic solution containing:
- NaCI in concentrations of over 0.2 M;
- sodium citrate in concentrations of over 0.05 M;
- an appropriate antibacterial agent, in suitable concentrations; and in keeping the said suspension under agitation at a temperature ranging from 2 to 8°C for a period ranging from 2 hours to 4 days.

13. Process according to Claim 1, **characterized in that**, in step (2), said chemical inactivation is obtained by treating the micro-organisms in an aqueous solution of a suitable disinfectant substance, at a concentration of 0.05-0.2 mg/l, for a period of 2-12 hours.

14. Process according to Claim 1, **characterized in that**, in step (2), said physical inactivation is obtained by exposure to UV rays or by heating to 55-65°C.

15. Process according to Claim 1, **characterized in that**, in step (3), the inactivated micro-organisms obtained in step (1) or (2) are suspended in a hypotonic aqueous solution containing the said digestive enzymes, for a period of time of between 4 hours and 4 days, at a temperature ranging from 2 to 8°C.

16. Process according to Claim 15, **characterized in that** said hypotonic aqueous solution further contains:
- NaCl in concentrations of less than 0.12 M;
- sodium citrate in concentrations of less than 0.025 M;
- an appropriate antibacterial agent in suitable concentrations.

17. Process according to Claim 1, **characterized in that**, in step (3), said essential nutrients are vitamins and/or glucides.

18. Process according to Claim 1, **characterized in that**, in step (3), said endogenous material that extracted in step (1) is first dialysed.

19. Process according to Claim 1, **characterized in that** the micro-organisms obtained in step (3) are concentrated and/or desiccated.

20. Process according to anyone of claims 1-19 further comprising a step of formulation into food composition for human consumption or for veterinary purposes.

## Patentansprüche

1. Verfahren zur Herstellung inaktivierter Mikroorganismen, die ein oder mehrere Verdauungsenzyme enthalten, das die folgenden Schritte umfasst:
(1) Extraktion der endozellulären Masse der Zellen eines geeigneten Mikroorganismus mit Hilfe einer hyperosmotischen Behandlung und Trennung der extrahierten endozellulären Masse;
(2) mögliche Inaktivierung des in Schritt (1) erhaltenen Mikroorganismus mittels chemischer oder physikalischer Mittel, die die äussere Membran des Mikroorganismus unverändert lassen;
(3) intrazelluläre Insertion von einem oder mehreren Verdauungsenzymen in dem in den Schritten (1) oder (2) erhaltenen Mikroorganismus mit Hilfe einer hypoosmotischen Behandlung, durch die Behandlung dieses Mikroorganismus in einer hypotonischen Suspension, enthaltend das endogene Material, das in Schritt (1) extrahiert wurde, zu dem möglicherweise Verdauungsenzyme anderer Art und weitere essentielle Nährstoffe hinzugegeben werden, oder eine geeignete hypotonische wässrige Suspension, die diese Verdauungsenzyme und andere wesentliche Nährstoffe enthält.

2. Verfahren gemäss Anspruch 1, **dadurch gekennzeichnet, dass** dieser Mikroorganismus kein Produzent von Verdauungsenzym ist, sondern als Vehikel dieser Enzyme dient.

3. Verfahren gemäss Anspruch 2, **dadurch gekennzeichnet, dass** diese Mikroorganismen Saccharomyces cerevisiae sind.

4. Verfahren gemäss Anspruch 1, **dadurch gekennzeichnet, dass** dieser Mikroorganismus ein Produzent von einem oder mehreren spezifischen Verdauungsenzymen ist, die aus der Gruppe ausgewählt werden, die aus Amylasen, Saccharidasen, Lipasen, Proteasen, Pectinasen, Cellulasen und Peptidasen besteht.

5. Verfahren gemäss Anspruch 4, **dadurch gekennzeichnet, dass** dieser Mikroorganismus zu den Spezies Acinetobacter, Bacillus, Aspergillus, Mikrococcus oder Endomyces gehört, wenn die Verdauungsenzyme Amylasen oder Saccharidasen sind.

6. Verfahren gemäss Anspruch 4, **dadurch gekennzeichnet, dass** dieser Mikroorganismus zu den Spezies Lactobacillus, Mikrococcus, Aspergillus, Pseudomonas oder Penicillium gehört, wenn die Enzyme Lipasen sind.

7. Verfahren gemäss Anspruch 4, **dadurch gekennzeichnet, dass**, wenn diese Enzyme Proteasen sind, der Mikroorganismus zu den Spezies Pseudomonas, Streptomyces, Bacillus oder Aspergillus gehört.

8. Verfahren gemäss Anspruch 4, **dadurch gekennzeichnet, dass** dieser Mikroorganismus zu den Spezies Acrocylindrium, Aspergillus, Penicillium, Fusarium, Clostridium, Bacillus oder Pseudomonas gehört, wenn die Enzyme Pectinasen sind.

9. Verfahren gemäss Anspruch 4, **dadurch gekennzeichnet, dass** dieser Mikroorganismus ein Bakterium der Spezies Ruminococcus, Cellulomonas oder Pseudomonas oder aber ein Pilz der Spezies Penicillium, Aspergillus oder Trichoderma oder aber ein Actinomycet der Spezies Streptomyces oder Thermoactinomyces ist, wenn die Verdauungsenzyme Cellulasen sind.

10. Verfahren gemäss Anspruch 1, **dadurch gekennzeichnet, dass** der Mikroorganismus, der kein Produzent von Verdauungsenzymen ist, aus menschlichem Fäkalmaterial oder Pansen-, Intestinal- und/oder Fäkalmaterial von Tieren stammt und entsprechend zur Produktion dieser Enzyme konditioniert ist.

11. Verfahren gemäss Anspruch 1, **dadurch gekennzeichnet, dass** die Mikroorganismen vor dem Schritt (1) durch Fermentierung zum Wachsen gebracht werden, und möglicherweise dem Gewinnen der Verdauungsenzyme, die während der Fermentierung hergestellt wurden.

12. Verfahren gemäss Anspruch 1, **dadurch gekennzeichnet, dass** die hyperosmotische Behandlung aus dem Suspendieren der Mikroorganismen in einer hypertonische Lösung besteht, die enthält:
- NaCl in Konzentrationen von über 0,2 M;
- Natriumcitrat in Konzentrationen von über 0,05 M;
- ein geeignetes antibakterielles Mittel in geeigneten Konzentrationen; und dem Bewahren dieser Suspension unter Rühren bei einer Temperatur, die von 2 bis 8°C reicht, für eine Dauer, die von 2 Stunden bis 4 Tagen reicht.

13. Verfahren gemäss Anspruch 1, **dadurch gekennzeichnet, dass** die chemische Inaktivierung in Schritt (2) durch die Behandlung der Mikroorganismen in einer wässrigen Lösung einer geeigneten Desinfektionsmittelsubstanz bei einer Konzentration von 0,05 bis 0,2 mg/l für eine Dauer von 2 bis 12 Stunden erreicht wird.

14. Verfahren gemäss Anspruch 1, **dadurch gekennzeichnet, dass** die physikalische Inaktivierung in Schritt (2) durch die Exposition gegenüber UV-Strahlen oder durch Erwärmen auf 55 bis 65°C erreicht wird.

15. Verfahren gemäss Anspruch 1, **dadurch gekennzeichnet, dass** die inaktivierten Mikroorganismen, die in Schritten (1) oder (2) erhalten wurden, in Schritt (3) in einer hypotonischen wässrigen Lösung, die die Verdauungsenzyme enthält, über eine Dauer von zwischen 4 Stunden und 4 Tagen bei einer Temperatur, die von 2 bis 8°C reicht, suspendiert werden.

16. Verfahren gemäss Anspruch 15, **dadurch gekennzeichnet, dass** die hypotonische wässrige Lösung ausserdem enthält:
- NaCl in einer Konzentration von weniger als 0,12 M;
- Natriumcitrat in einer Konzentration von weniger als 0,025 M;
- ein geeignetes antibakterielles Mittel in passenden Konzentrationen.

17. Verfahren gemäss Anspruch 1, **dadurch gekennzeichnet, dass** die essentiellen Nährstoffe in Schritt (3) Vitamine und/oder Glucide sind.

18. Verfahren gemäss Anspruch 1, **dadurch gekennzeichnet, dass** das endogene Material in Schritt (3), das in Schritt (1) extrahiert wurde, zuerst dialysiert wird.

19. Verfahren gemäss Anspruch 1, **dadurch gekennzeichnet, dass** die in Schritt 3 erhaltenen Mikroorganismen konzentriert und/oder getrocknet werden.

20. Verfahren gemäss einem der Ansprüche 1 bis 19, das ausserdem einen Schritt der Formulierung in eine Futterzusammensetzung für den menschlichen Verbrauch oder für veterinäre Zwecke umfasst.

## Revendications

1. Procédé de production de micro-organismes inactivés contenant une ou plusieurs enzymes digestives, comprenant les étapes suivantes :
1) extraction de la masse endocellulaire des cellules d'un micro-organisme approprié à l'aide d'un traitement hyperosmotique, et séparation de la masse endocellulaire extraite ;
2) inactivation possible du micro-organisme obtenu à l'étape (1) par un processus chimique ou physique, n'altérant pas la membrane externe du micro-organisme;
3) insertion intracellulaire d'une ou plusieurs enzymes digestives dans le micro-organisme inactivé obtenu à l'étape (1) ou (2) au moyen d'un traitement hypo-osmotique, en traitant ledit micro-organisme dans une suspension hypotonique contenant le matériau endogène extrait à l'étape (1), auquel ont été éventuellement ajoutés des enzymes digestives d'une autre nature et d'autres nutriments essentiels, ou une suspension aqueuse hypotonique adaptée contenant lesdites enzymes digestives et d'autres nutriments essentiels.

2. Procédé selon la revendication 1, **caractérisé en ce que** ledit micro-organisme n'est pas producteur des enzymes digestives, mais agit comme un véhicule desdites enzymes.

3. Procédé selon la revendication 2, **caractérisé en ce que** ledit micro-organisme est *Saccharomyces cerevisiae.*

4. Procédé selon la revendication 1, **caractérisé en ce que** ledit micro-organisme est producteur d'une ou plusieurs enzymes digestives spécifiques choisies dans le groupe comprenant les amylases, les saccharidases, les lipases, les protéases, les pectinases, les cellulases et les peptidases.

5. Procédé selon la revendication 4, **caractérisé en ce que** lesdites enzymes digestives sont des amylases ou des saccharidases, ledit micro-organisme appartient à l'espèce *Acinetobacter, Bacillus, Aspergillus, Micrococcus* ou *Endomyces.*

6. Procédé selon la revendication 4, **caractérisé en ce que** lesdites enzymes digestives sont des lipases, ledit micro-organisme appartient à l'espèce *Lactobacillus, Micrococcus, Aspergillus, Pseudomonas* ou *Penicillium.*

7. Procédé selon la revendication 4, **caractérisé en ce que**, lorsque lesdites enzymes digestives sont des protéases, ledit micro-organisme appartient à l'espèce *Pseudomonas, Streptomyces, Bacillus* ou *Aspergillus.*

8. Procédé selon la revendication 4, **caractérisé en ce que**, lorsque lesdites enzymes digestives sont des pectinases, ledit micro-organisme appartient à l'espèce *Acrocylindrum, Aspergillus, Penicillium, Fusarium, Clostridium, Bacillus* ou *Pseudomonas.*

9. Procédé selon la revendication 4, **caractérisé en ce que**, lorsque lesdites enzymes digestives sont des cellulases, ledit micro-organisme est une bactérie de l'espèce *Ruminococcus, Cellulomonas ou Pseudomonas,* ou également un champignon de l'espèce *Penicillium, Aspergillus* ou *Trichoderma,* ou encore un actinomycète de l'espèce *Streptomyces* ou *Thermoactinomyces.*

10. Procédé selon la revendication 1, **caractérisé en ce que** ledit micro-organisme n'est pas producteur d'enzymes digestives, est isolé des matières fécales humaines ou ruminales, intestinales et/ou fécales des animaux de bétail, et est conditionné de façon appropriée pour la production desdites enzymes.

11. Procédé selon la revendication 1, **caractérisé en ce que**, avant l'étape (1), lesdits micro-organismes sont fabriqués pour se développer par fermentation, en récupérant éventuellement les enzymes digestives produites durant ladite fermentation.

12. Procédé selon la revendication 1, **caractérisé en ce que** ledit traitement hyperosmotique consiste à mettre ledit micro-organisme en suspension dans une solution hypertonique contenant :
- NaC1 dans des concentrations supérieures à 0,2 M ;
- du citrate de sodium dans des concentrations supérieures à 0,05 M ;
- un agent antibactérien approprié, dans des concentrations adaptées ;
et à garder ladite suspension sous agitation à une température située entre 2 et 8°C pendant une période allant de 2 heures à 4 jours.

13. Procédé selon la revendication 1, **caractérisé en ce que**, à l'étape (2), ladite inactivation chimique est obtenue en traitant les micro-organismes dans une solution aqueuse d'une substance désinfectante adaptée, avec une concentration de 0,05 à 0,2 mg/l, pendant une période de 2 à 12 heures.

14. Procédé selon la revendication 1, **caractérisé en ce que**, à l'étape (2), ladite inactivation physique est obtenue par exposition aux rayons UV ou par un réchauffement à 55-65°C.

15. Procédé selon la revendication 1, **caractérisé en ce que**, à l'étape (3), les micro-organismes inactivés obtenus à l'étape (1) ou (2) sont mis en suspension dans une solution aqueuse hypotonique contenant lesdites enzymes digestives, pendant une période comprise entre 4 heures et 4 jours, à une température située entre 2 et 8°C.

16. Procédé selon la revendication 15, **caractérisé en ce que** ladite solution aqueuse hypotonique contient en outre :
- NaCl dans des concentrations inférieures à 0,12 M ;
- du citrate de sodium dans des concentrations inférieures à 0,025 M ;
- un agent antibactérien approprié dans des concentrations adaptées.

17. Procédé selon la revendication 1, **caractérisé en ce que**, à l'étape (3), lesdits nutriments essentiels sont des vitamines et/ou des glucides.

18. Procédé selon la revendication 1, **caractérisé en ce que**, à l'étape (3), ledit matériau endogène extrait à l'étape (1) est tout d'abord dialysé.

19. Procédé selon la revendication 1, **caractérisé en ce que** les micro-organismes obtenus à l'étape (3) sont concentrés et/ou desséchés.

20. Procédé selon l'une quelconque des revendications 1 à 19, comprenant en outre une étape de formulation en une composition alimentaire destinée à la consommation humaine ou à des fins vétérinaires.
